Europäisches Patentamt

European Patent Office

Office européen des brevets

(19)

(11) Numéro de publication: **0 052 049**
**B1**

(12) # FASCICULE DE BREVET EUROPEEN

(45) Date de publication du fascicule du brevet:
**18.04.84**

(51) Int. Cl.³: **C 12 M 1/00**

(21) Numéro de dépôt: **81401723.2**

(22) Date de dépôt: **28.10.81**

(54) **Procédé de production de méthane par fermentation de déchets et installation pour la mise en oeuvre de ce procédé.**

(30) Priorité: **07.11.80 FR 8023861**

(43) Date de publication de la demande:
**19.05.82 Bulletin 82/20**

(45) Mention de la délivrance du brevet:
**18.04.84 Bulletin 84/16**

(84) Etats contractants désignés:
**AT BE CH DE GB IT LI LU NL SE**

(56) Documents cités:
**DE - C - 884 176**
**DE - C - 905 598**
**FR - A - 893 537**
**FR - A - 914 808**
**FR - A - 1 009 247**
**FR - A - 2 305 113**
**US - A - 3 934 999**

(73) Titulaire: **Pons, Gérard Antoine Justin, 18-20 rue Marc Sangnier, F-64000 Pau (FR)**

(72) Inventeur: **Pons, Gérard Antoine Justin, 18-20 rue Marc Sangnier, F-64000 Pau (FR)**

(74) Mandataire: **Weinstein, Zinovi et al, Cabinet Z. WEINSTEIN 20, Avenue de Friedland, F-75008 Paris (FR)**

# Procédé de production de méthane par fermentation de déchets et installation pour la mise en œuvre de ce procédé

La présente invention a essentiellement pour objet un procédé de production de méthane à partir de déchets végétaux et/ou animaux divers.

Elle vise également une installation comportant application de ce procédé.

On sait qu'actuellement il y a un regain d'intérêt pour les sources d'énergie de remplacement parmi lesquelles la fermentation de déchets organiques divers en vue de produire du méthane tient à l'heure actuelle une place prépondérante. On a d'ailleurs déjà proposé un certain nombre d'installations permettant de produire du méthane ou biogaz à partir de deux procédés, à savoir le procédé continu ou le procédé discontinu.

Brièvement parlant, le procédé discontinu exige des manipulations pénibles ainsi que la prévision de plusieurs cuves de fermentation ou digesteurs. En outre, lorsque les manipulations sont mécaniques, elles impliquent de larges ouvertures sur les digesteurs, donc des problèmes d'étanchéité et des pertes caloriques importantes lors de la vidange des digesteurs.

Quant au procédé continu, une part importante du méthane produit doit servir à réchauffer le digesteur, ce qui conduit à une production nette de biogaz qui demeure relativement faible. De plus, dans un tel procédé, on se heurte bien souvent à des problèmes particuliers, tels que par exemple des problèmes d'étanchéité et d'équilibrage de la fermentation.

Eu égard à ces deux types de procédé, on citera par exemple le brevet français n° 1 009 247 et le brevet allemand n° 884 176 concernant tous deux des procédés de fermentation qui, par ailleurs, ne permettent pas de rompre la croûte se formant nécessairement à la surface du liquide de fermentation au cours du processus de digestion.

D'une manière générale, la présente invention a pour but de remédier aux inconvénients précités en proposant un procédé et une installation de production de méthane qui réunissent les avantages découlant de l'utilisation des procédés continu et discontinu, tout en supprimant les inconvénients particuliers de chaque procédé, ce qui conduit à un haut rendement de la production en biogaz.

Plus précisément, le procédé et l'installation selon l'invention permettent de façon quasi-continue et sans manipulations manuelles à l'intérieur du digesteur, la mise en fermentation de tous déchets végétaux et/ou animaux, liquides et/ou solides, de façon à réaliser une fermentation à haut rendement en biogaz ainsi qu'une épuration parfaite des effluents.

A cet effet, l'invention a pour objet un procédé de production de méthane à partir de déchets végétaux et/ou animaux sous forme liquide et/ou solide, et du type consistant à disposer lesdits déchets dans au moins un conteneur où ils subissent une fermentation aérobie avant d'être plongés et déplacés à l'intérieur d'une enceinte à fermentation anaérobie contenant un liquide de fermentation méthanique, caractérisé en ce qu'au moins la partie supérieure du conteneur est maintenue en affleurement avec le niveau du liquide précité de fermentation méthanique pendant au moins une partie du trajet des conteneurs dans l'enceinte à fermentation anaérobie.

Un tel affleurement du conteneur en mouvement dans l'enceinte anaérobie évite avantageusement la formation d'une croûte à la surface du liquide de fermentation, ou bien détruit ladite croûte si celle-ci présente un début de formation. Cela représente un avantage considérable car on sait que, dans les procédés actuellement connus, la présence d'une telle croûte est pratiquement obligatoire et peut aller jusqu'à provoquer l'explosion de l'enceinte à liquide de fermentation.

L'invention vise également une installation pour la mise en œuvre du procédé ci-dessus et du type comprenant une cuve susceptible de recevoir des déchets végétaux et/ou animaux préalablement disposés dans au moins un conteneur et subissant dans un compartiment de cette cuve une fermentation aérobie suivie par une fermentation anaérobie dans un autre compartiment où se déplacent lesdits conteneurs, caractérisée en ce que le compartiment de fermentation anaérobie est intérieurement muni d'un convoyeur d'entraînement des conteneurs vers une position d'affleurement avec la surface du liquide de fermentation méthanique contenu dans ledit compartiment.

Suivant une autre caractéristique de cette installation, dans le cas où les conteneurs sont des caissons parallélépipèdiques, le convoyeur précité est constitué par une chaîne associée à une rampe sinueuse qui s'étend tant dans le compartiment précité que dans le compartiment d'évacuation des caissons de la cuve.

Selon encore une autre caractéristique de l'invention, les caissons sont pourvus sur deux faces opposées d'ergots venant en prise sur la chaîne et guidés dans des rainures ou glissières prévues sur les parois des compartiments de fermentation aérobie et anaérobie.

Selon encore une autre caractéristique de l'invention, des rampes d'arrosage de la surface du liquide précité sont prévues entre ladite surface et la paroi supérieure du compartiment de fermentation anaérobie.

Suivant un autre mode de réalisation, dans le cas où les conteneurs sont des sacs ajourés en matière plastique, un convoyeur de transport de ces sacs est agencé à la partie inférieure du compartiment précité, tandis qu'à la partie supérieure de ce compartiment est prévue une grille retenant les sacs en affleurement avec la surface du liquide de fermentation et formant une rampe d'arrosage.

Avantageusement, la grille précitée peut être remplacée par un convoyeur retenant et transportant les sacs en affleurement avec la surface du liquide de fermentation, tandis qu'une rampe d'arrosage est prévue au dessus dudit convoyeur.

On ajoutera encore ici qu'une alimentation en liquide, pulsée ou sous forte pression débouche dans la paroi de fond du compartiment de fermentation anaérobie pour solliciter les sacs vers le haut de ce compartiment.

On ajoutera encore que, quel que soit le mode de conditionnement des déchets, sacs ou caissons, les rampes d'arrosage et la grille précitées sont alimentées par un circuit en liquide de fermentation méthanique contenu dans le compartiment anaérobie et mélangé à du lisier contenu dans une fosse.

D'autres caractéristiques et avantages de l'invention apparaîtront mieux dans la description détaillée qui suit et se réfère aux dessins annexés, donnés uniquement à titre d'exemples, et dans lesquels:

La fig. 1 est une vue schématique et en élévation d'un premier mode de réalisation d'une installation conforme aux principes de l'invention, et utilisant des déchets animaux et/ou végétaux préalablement conditionnés en caissons.

La fig. 2 est une vue schématique et en élévation d'un autre mode de réalisation utilisant des déchets conditionnés en sacs ajourés ou perforés en matière plastique.

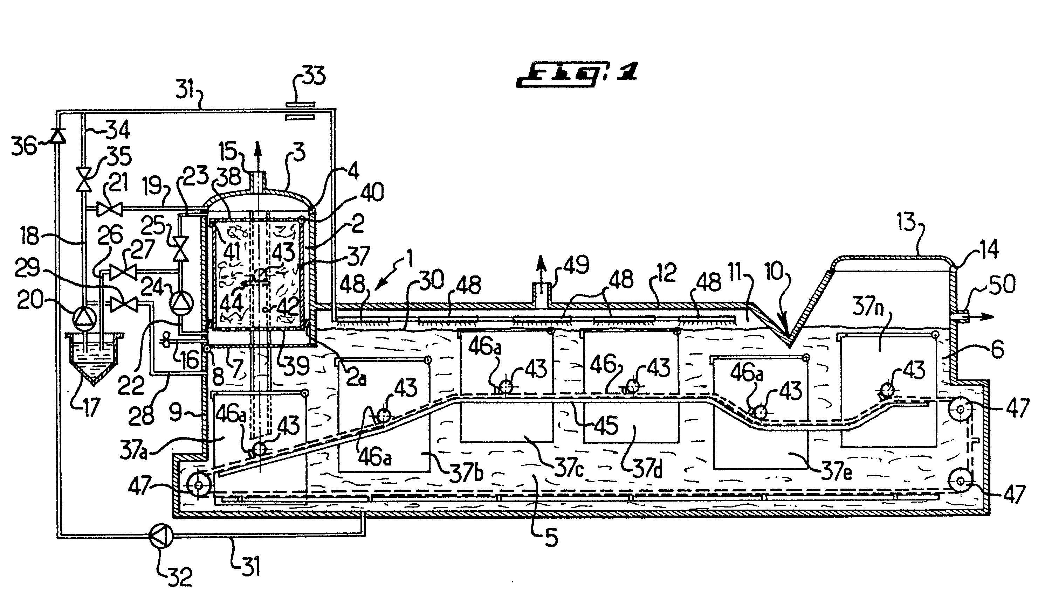

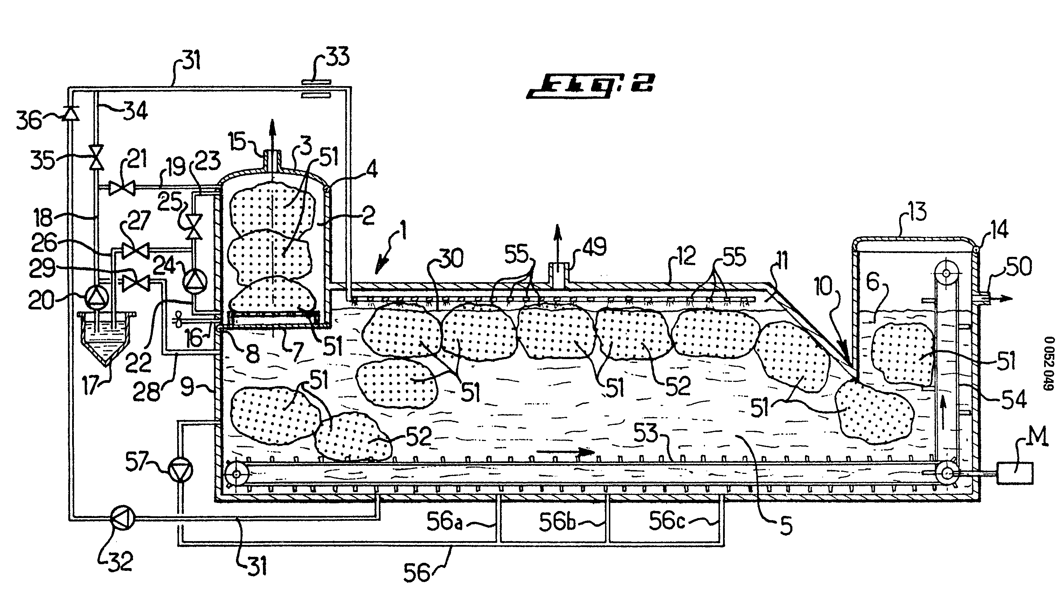

En se reportant aux fig. 1 et 2, on voit qu'une installation conforme à l'invention comprend essentiellement un digesteur 1 formant une cuve unique qui se compose essentiellement de trois parties, à savoir: un compartiment supérieur 2 formant enceinte de fermentation aérobie et accessible depuis l'extérieur par une porte ou analogue 3 articulée en 4; un compartiment inférieur 5 en forme de couloir constituant l'enceinte de fermentation anaérobie et s'étendant depuis la partie basse du compartiment aérobie 2 tout en communiquant avec lui comme on le verra plus loin; et un compartiment 6 d'évacuation des déchets et des effluents, lequel compartiment constitue l'extrémité du couloir 5 et communique avec lui.

On a montré en 7 une porte, trappe ou analogue, articulée en 8 sur la paroi latérale 9 du digesteur 1 et permettant la communication du compartiment supérieur aérobie 2 avec le compartiment ou couloir inférieur anaérobie 5. Ce dernier communique avec le compartiment d'évacuation 6 par l'intermédiaire d'une partie formant sas 10 assurant l'isolement du biogaz ou méthane produit en 11, vis-à-vis de l'atmosphère extérieure. Cette partie formant sas 10 est, suivant un exemple de réalisation, tout simplement réalisée par un infléchissement vers le bas de la paroi supérieure 12 du compartiment 5 du digesteur 1.

Le compartiment d'évacuation 6 comporte à son sommet une porte d'accès 13, par exemple articulée en 14 sur la paroi du digesteur 1, étant bien entendu que cette porte présente les qualités d'étanchéité requises.

La porte ou trappe 7 entre le compartiment supérieur 2 et le compartiment inférieur 5 qui renferme le liquide de fermentation méthanique, présente elle aussi toutes les qualités d'étanchéité requises. Cela est également le cas de la porte 3 au sommet du compartiment 2 et qui comporte une évacuation 15 des gaz de la fermentation aérobie.

La fermentation aérobie dans le compartiment 2 est réalisée par le circuit visible sur la gauche des fig. 1 et 2 et que l'on décrira succintement ci-après: une introduction d'air, au moyen d'un ventilateur par exemple, par une conduite 16 débouchant à la base du compartiment 2 obturable par la porte 7; une alimentation en lisier ou analogue contenu dans une fosse 17 par des conduites 18 et 19 qui débouchent à la partie supérieure du compartiment 2 et sont susceptibles d'arroser en pluie les déchets qui y sont contenus, la conduite 18 étant équipée d'une pompe 20 et d'une vanne 21; un circuit de retour 22, 23 du lisier depuis le bas, vers le haut du compartiment aérobie 2, ce circuit comprenant une pompe 24 et une vanne 25; un conduit 26 équipé d'une vanne 27 et permettant le retour à la fosse 17 de l'excès du lisier liquide au fond du compartiment aérobie 2; et une conduite 28 avec vanne 29, raccordée au circuit d'alimentation 18, 19 et permettant une alimentation et/ou un appoint de lisier frais dans le compartiment inférieur ou couloir 5 contenant le liquide de fermentation méthanique et dont on a montré le niveau en 30.

Par ailleurs, on a montré schématiquement en 31 une conduite avec pompe 32 et système éventuel de chauffage 33, laquelle conduite renvoie le liquide contenu dans le compartiment 5 vers le haut de ce compartiment entre la paroi supérieure 12 et la surface 30 du liquide de façon à arroser en pluie ladite surface. Cet arrosage peut être également réalisé avec un mélange de liquide et de lisier frais provenant de la fosse 18 grâce au conduit 34 équipé d'une vanne 35 et raccordant le circuit d'alimentation 18 à la conduite 31. Bien entendu, dans ce cas là, un clapet anti-retour 36 sera prévu sur la conduite 31.

Toutes les dispositions ci-dessus s'appliquent aux deux modes de réalisation visibles respectivement sur les fig. 1 et 2.

On se reportera maintenant plus particulièrement à la fig. 1 dans laquelle le digesteur 1 est destiné à traiter des déchets organiques solides ou semi-solides tels que par exemple fumier pailleux, boues épaisses de lisier, déchets végétaux, ordures ménagères, etc., contenus dans de caissons parallélépipèdiques 37 dont les faces supérieure 38 et inférieure 39 sont constituées par des grilles, au moins la grille 38 étant articulée et formant porte d'accès audit caisson 37. C'est ainsi que par exemple la grille 38 peut être articulée en 40 sur la paroi du caisson et comporter des moyens de fermeture appropriés montrés schématiquement en 41.

Comme on l'a montré schématiquement sur la fig. 1, on a prévu sur les parois du compartiment supérieur 2 et inférieur 5 du digesteur 1 des rainures ou analogues 42 dans lesquelles coulissent des ergots ou analogues 43 prévus sur deux faces opposées du caisson 37. On a montré en 44 une butée ou analogue déverrouillable prévue dans les rainures 42 et servant à maintenir le caisson 37 dans le compartiment supérieur 2. Ce dispositif peut être d'une structure quelconque et commandé d'une manière quelconque, sans sortir du cadre de l'invention. On notera encore ici que le caisson 37 comporte sur sa périphérie un anneau de guidage 2a du caisson dans le compartiment 2 et également d'étanchéité dudit caisson vis-à-vis des parois du compartiment 2.

A l'intérieur du compartiment anaérobie 5, est prévue une rampe ou analogue 45 suivant un trajet sinueux et à laquelle est associée une chaîne ou bande sans fin 46 passant autour de rouleaux, poulies ou analogues 47 de manière à réaliser un convoyeur de transport des caissons 37 comme on le décrira en détail ultérieurement. On notera cependant ici que la chaîne 46 comprend des moyens appropriés et montrés schématiquement en 46a pour recevoir et entraîner les ergots 43 du caisson 37.

Entre la paroi supérieure 12 du compartiment 5 et la surface 30 du liquide de fermentation méthanique contenu dans ledit compartiment, on prévoit des rampes 48 d'arrosage en pluie de la surface 30, lesquelles rampes sont raccordées à la conduite 31 décrite précédemment.

Bien entendu, la paroi supérieure 12 comporte une évacuation 49 du méthane produit, et il est également prévu sur la paroi du compartiment d'évacuation 6 une sortie 50 des effluents qui sont par exemple envoyés dans des bassins pour pouvoir être utilisés par la suite.

D'ailleurs, les éléments précités 49 et 50 se retrouvent dans le mode de réalisation d'installation visible sur la fig. 2 et qui sera maintenant décrite en plus de détails.

Cette installation est destinée à traiter des déchets divers, comme on l'a dit précédemment qui ont été préalablement conditionnés dans des sacs en matière plastique ajourés et comportant de préférence des perforations multiples. Ces sacs remplis de déchets ont été montrés schématiquement en 51 sur la fig. 2, et l'on voit également en 52 les perforations de ces sacs.

A la partie inférieure du compoartiment ou couloir de fermentation anaérobie 5, est prévu un convoyeur 53 susceptible d'entraîner lesdits sacs depuis la gauche vers la droite du compartiment 5, lequel convoyeur peut être entraîné par un moteur quelconque M. Ce même moteur peut être utilisé pour entraîner un autre convoyeur ou élévateur 54 des sacs 51 dans le compartiment d'évacuation 6 des sacs.

Entre la paroi supérieure 12 du compartiment 5 et la surface 30 du liquide de fermentation, on prévoit une grille ou analogue 55 permettant la retenue des sacs 51 sensiblement en affleurement avec la surface du liquide 30. Suivant une réalisation préférée, la grille 55 peut avantageusement constituer plusieurs rampes d'arrosage de la surface 30 en liquide de fermentation et/ou lisier frais ou analogue, ladite grille étant raccordée à la conduite 31, comme expliqué précédemment.

Il est important de noter ici que la grille précitée 55 peut être omise et remplacée par un autre convoyeur tel que 53 qui assura un double rôle et à savoir un rôle de maintien des sacs 51 en affleurement avec le niveau 30, et un rôle de transport desdits sacs vers l'évacuation 6. Dans un tel cas, on prévoiera une rampe d'arrosage en liquide et/ou lisier frais au-dessus du convoyeur en question.

Enfin, on prévoit à la partie inférieure du compartiment anaérobie 5 une alimentation 56 en liquide, pulsée ou sous forte pression, grâce à une pompe 57, cette alimentation se ramifiant en 56a, 56b, 56c, etc. pour déboucher dans la paroi de fond du compartiment 5. Ceci permet aux sacs 51 plus ou moins lourds d'être brassés et sollicités par un courant de liquide vers le haut du compartiment 5 au fur et à mesure de leur déplacement provoqué par le convoyeur 53.

On décrira maintenant le fonctionnement de l'installation de la fig. 2, lequel, dans son principe, est voisin de celui de la fig. 1, qui sera décrit par la suite.

Les sacs 51 en matière plastique perforés sont préalablement remplis de déchets éventuellement broyés qui peuvent être constitués par de la paille, de l'herbe, ou même des ordures ménagères autres que verre, ferraille et plastique, avec éventuellement des boues de lisier provenant des bâtiments d'élevage. Ces sacs sont introduits par la porte 3 dans le compartiment à fermentation aérobie 2 et dans lequel les déchets contenus dans les sacs sont imbibés de lisier ou analogue provenant du circuit 19 et éventuellement recyclé par le circuit 22, 23. Ensuite, les déchets sont aérés par insufflation d'air provenant de la conduite 16 et on surveille la température du compartiment 2 jusqu'à ce qu'elle atteigne environ 55-60°C. La porte inférieure ou trappe 7 formant le fond du compartiment 2 est alors ouverte par un moyen quelconque et les sacs de déchets plongent dans le compartiment ou couloir anaérobie 5. On notera ici que les sacs 51 peuvent flotter plus ou moins dans le liquide de fermentation méthanique et ils peuvent être préalablement lestés si on le désire de façon à aboutir sur le convoyeur 53 qui les entraîne. Au fur et à mesure de la fermentation des déchets contenus dans les sacs, la densité peut changer et les sacs peuvent remonter à la surface 30 du liquide. A ce stade, ils sont maintenus par la grille 55 ou par un convoyeur tel que 53 au niveau de la surface 30 du liquide, ce qui contribue très avantageusement à éviter la formation d'une croûte sur ladite surface ou à détruire la croûte qui aurait tendance à s'y former. Pendant le déplacement des sacs dans le couloir 5, le liquide de fermentation sera brassé en permanence par le circuit 56 ce qui contribuera non seulement à agiter les sacs pour favoriser la fermentation aboutissant à la production de méthane 11 sortant par l'évacuation 49, mais encore à éviter la formation de la croûte à la surface 30 du liquide. Ainsi, le compartiment anaérobie 5 fonctionne comme un système à la fois continu et discontinu dans la mesure où chaque sac en mouvement constitue une zone de fermentation autonome.

Arrivés en bout du couloir 5, les sacs 51 sont automatiquement acheminés vers le compartiment d'évacuation 6 grâce à la paroi infléchie du sas 10, et ils sont dirigés vers l'élévateur 54 qui permet leur extraction du digesteur 1 par la porte 13.

On notera ici que les déchets peuvent être préalablement préparés en cubes ou analogues de façon à être introduits par la suite dans les sacs 51 en utilisant des moyens mécaniques quelconques. En tout cas, on voit qu'il n'y a aucune manipulation des sacs 51 lorsqu'ils sont placés et se meuvent dans le digesteur 1.

L'installation de la fig. 1 fonctionne suivant le même principe général que ci-dessus, sauf que l'utilisation des caissons 37 est faite à la place des sacs 51.

On introduit le caisson 37 préalablement chargé de déchets, végétaux ou autres dans le compartiment à fermentation aérobie 2. Le caisson y est maintenu, par le fait que les ergots 43 butent sur le dispositif d'arrêt déblocable 44. Ensuite, les mêmes opérations que précédemment, à savoir imbibation et aération des déchets dans le caisson, sont réalisées, le liquide passant à travers les grilles 38 et 39 du caisson et étant éventuellement recyclé et débarrassé du liquide en excès, comme on l'a décrit précédemment.

Puis la porte 7 est ouverte et, par un moyen de commande quelconque, on déverrouille la butée 44, ce qui provoque la chute par gravité du caisson 37 qui plonge dans le liquide du compartiment anaérobie 5, lequel utilise évidemment et avantageusement la chaleur résultant de la fermentation aérobie dans le compartiment 2.

Le caisson tombe sur le brin supérieur du convoyeur 46 porté par la rampe 45 et est entraîné vers la droite en suivant un trajet sinueux, de façon que la partie supérieure du caisson affleure le niveau 30 du liquide dans le compartiment 5 en vue d'éviter la formation d'une croûte, comme on l'a déjà expliqué. Comme on le voit clairement sur la fig. 1, le caisson prend les positions successives 37a, 37b, 37c, 37d, 37e, etc. pour aboutir en 37n au compartiment d'évacuation 6 ou il peut être extrait à l'aide d'un engin de levage quelconque qui le soulève par les ergots 43.

Comme pour le mode de réalisation de la fig. 2, le digesteur 1 fonctionne dans son ensemble comme un système continu, tandis que les caissons 37 fonctionnent comme une série de digesteurs discontinus, mais sur lesquels on n'a pas à intervenir après qu'ils aient été introduits dans le digesteur. De plus, on notera ici que les caissons extraits du compartiment 6 peuvent être rechargés en déchets et réintroduits dans le compartiment aérobie 2, de sorte que le digesteur 1 est continuellement rempli de caissons 37 qui s'y déplacent de manière continue ou pas-à-pas de façon à obtenir le meilleur rendement en méthane produit et recueilli en 49.

On a donc réalisé suivant l'invention un digesteur qui supprime toutes les manipulations manuelles en les remplaçant par des manipulations mécaniques et automatiques, qui permet l'utilisation directe des apports calorifiques résultant de la fermentation aérobie et qui permet d'obtenir une production continue et élevée de biogaz.

Bien entendu l'invention n'est nullement limitée aux modes de réalisation décrits et illustrés qui n'ont été donnés qu'à titre d'exemple.

C'est ainsi que le système de verrouillage du caisson dans le compartiment aérobie, l'articulation et la commande des diverses trappes ou portes de l'installation et la structure des chaînes du convoyeur d'entraînement des caissons peuvent être quelconques, sans sortir du cadre de l'invention.

C'est dire que celle-ci comprend tous les équivalents techniques des moyens décrits, ainsi que leurs combinaisons si elles sont effectuées suivant son esprit et mises en œuvre dans le cadre de la protection revendiquée.

## Revendications

1. Procédé de production de méthane à partir de déchets végétaux et/ou animaux sous forme liquide et/ou solide, et du type consistant à disposer lesdits déchets dans au moins un conteneur où ils subissent une fermentation aérobie avant d'être plongés et déplacés à l'intérieur d'une enceinte à fermentation anaérobie contenant un liquide de fermentation méthanique caractérisé en ce qu'au moins la partie supérieure du conteneur est maintenue en affleurement avec le niveau du liquide précité de fermentation méthanique pendant au moins une partie du trajet des conteneurs dans l'enceinte à fermentation anaérobie.

2. Installation pour la mise en œuvre du procédé selon la revendication 1, et du type comprenant une cuve (1) susceptible de recevoir des déchets végétaux et/ou animaux préalablement disposés dans au moins un conteneur (37, 51,) et subissant dans un compartiment (2) de cette cuve une fermentation aérobie suivie par une fermentation anaérobie dans un autre compartiment (5) où se déplacent lesdits conteneurs, caractérisée en ce que le compartiment (5) de fermentation anaérobie est intérieurement muni d'un convoyeur (45, 46, 53) d'entraînement des conteneurs (37, 51) vers une position d'affleurement avec la surface (30) du liquide de fermentation méthanique contenu dans ledit compartiment (5).

3. Installation selon la revendication 2, caractérisée en ce que, dans le cas où les conteneurs sont des caissons parallélépipédiques (37), le convoyeur précité est constitué par une chaîne (46) associée à une rampe sinueuse (45) qui s'étend tant dans le compartiment précité (5) que dans le compartiment (6) d'évacuation des caissons de la cuve (1).

4. Installation selon la revendication 2 ou 3, caractérisée en ce que les caissons (37) sont pourvus sur deux faces opposées d'ergots (43) venant en prise sur la chaîne (46) et guidés dans des rainures ou glissières (42) prévues sur les parois des compartiments (2, 5) de fermentation aérobie et anaérobie.

5. Installation selon l'une des revendications 2 à 4, caractérisée en ce que des rampes (48) d'arrosage de la surface (30) du liquide précité sont prévues entre ladite surface et la paroi supérieure (12) du compartiment (5) de fermentation anaérobie.

6. Installation selon la revendication 2, caractérisée en ce que dans le cas où les conteneurs sont des sacs ajourés (51) en matière plastique, un convoyeur (53) de transport de ces sacs est agencé à la partie inférieure du compartiment précité (5), tandis qu'à la partie supérieure de ce compartiment est prévue une grille (55) retenant les sacs en affleurement avec la surface (30) du liquide de fermentation et formant une rampe d'arrosage.

7. Installation selon la revendication 6, caractérisée en ce que la grille précitée (55) peut être remplacée par un convoyeur retenant et transportant les sacs (51) en affleurement avec la surface (30) du liquide de fermentation, tandis qu'une rampe d'arrosage est prévue au-dessus dudit convoyeur.

8. Installation suivant la revendication 6 ou 7, caractérisée en ce qu'une alimentation (56) en liquide, pulsée ou sous forte pression, débouche dans la paroi de fond du compartiment (5) de fermentation anaérobie pour solliciter les sacs (51) vers le haut de ce compartiment.

9. Installation suivant l'une des revendications 2 à 8, caractérisée en ce que les rampes d'arrosage et la grille précitées sont alimentées par un circuit (31, 34) en liquide de fermentation méthanique contenu dans le compartiment (5) et mélangé à du lisier contenu dans une fosse (17).

## Patentansprüche

1. Verfahren zur Erzeugung von Methan aus Pflanzen- und/oder Tierabfällen in flüssiger und/oder fester Form, und derjenigen Gattung, die darin besteht, die besagten Abfälle in wenigstens einen Behälter zu tun, wo sie einer Aerobgärung unterworfen werden, bevor sie in einem eine methanische Gärungsflüssigkeit enthaltenden Raum zur Anaerobgärung getaucht und verschoben werden, dadurch gekennzeichnet, dass wenigstens der Oberteil des Behälters mit der Höhenlage der vorgenannten methanischen Gärungsflüssigkeit während wenigstens einem Teil des Weges der Behälter in dem umschlossenen Raum zur Anaerobgärung fluchtend bündig gehalten wird.

2. Anlage zur Durchführung des Verfahrens nach Anspruch 1 und der Gattung mit einem Gefäss (1) zur Aufnahme von Pflanzen- und/oder Tierabfällen, die vorher in wenigstens einen Behälter (37, 51) getan worden sind und in einem Abteil (2) dieses Gefässes eine Aerobgärung und

in einem anderen Abteil (5), wo sich die besagten Behälter bewegen, eine nachfolgende Anaerobgärung erfahren, dadurch gekennzeichnet, dass das Abteil (5) zur Anaerobgärung innerhalb mit einem Förderer (45, 46, 53) ausgestattet ist, zur Mitnahme der Behälter (37, 51) zu einer mit der Oberfläche (30) der in dem besagten Abteil (5) enthaltenen methanischen Gärungsflüssigkeit fluchtend bündigen Lage.

3. Anlage nach Anspruch 2, dadurch gekennzeichnet, dass in dem Fall, wo die Behälter quaderförmige Kasten (37) sind, der vorgenannte Förderer aus einer Kette (46) besteht, welche einer gewundenen Rampe (45) zugeordnet ist, die sich sowohl in dem vorgenannten Abteil (5) als auch in dem Abteil (6) zur Abfuhr der Kasten des Gefässes (1) erstreckt.

4. Anlage nach Anspruch 2 oder 3, dadurch gekennzeichnet, dass die Kasten (37) auf den beiden entgegengesetzten Seiten mit der Kette (46) in Eingriff kommenden und in an den Wandungen der Abteile (2, 5) zur Aerob- und Anaerobgärung vorgesehenen Nuten bzw. Gleitführungen (42) geführten Nocken (43) versehen sind.

5. Anlage nach einem der Ansprüche 2 bis 4, dadurch gekennzeichnet, dass Verteiler (48) zum Besprengen der Oberfläche (30) der vorgenannten Flüssigkeit zwischen der besagten Oberfläche und der oberen Wandung (12) des Abteils (5) zur Anaerobgärung vorgesehen sind.

6. Anlage nach Anspruch 2, dadurch gekennzeichnet, dass in dem Fall, wo die Behälter gelochte Säcke (51) aus Kunststoff sind, ein Förderer (53) zur Beförderung dieser Säcke an dem unteren Teil des vorgenannten Abteils (5) angeordnet ist, während an dem oberen Teil dieses Abteils ein die Säcke mit der Oberfläche (30) der Gärungsflüssigkeit fluchtend bündig zurückhaltendes und einen Besprengungsverteiler bildendes Gitter (55) vorgesehen ist.

7. Anlage nach Anspruch 6, dadurch gekennzeichnet, dass das vorgenannte Gitter (55) durch einen die Säcke (51) fluchtend bündig mit der Oberfläche (30) der Gärungsflüssigkeit zurückhaltenden und befördernden Förderer ersetzt werden kann, während ein Besprengungsverteiler über den besagten Förderer vorgesehen ist.

8. Anlage nach Anspruch 6 oder 7, dadurch gekennzeichnet, dass eine gepulste oder unter hohem Druck stehende Flüssigkeitsspeisevorrichtung (56) in die Bodenwandung des Abteils (5) zur Anaerobgärung mündet, um die Säcke (51) in diesem Abteil aufwärts zu treiben.

9. Anlage nach einem der Ansprüche 2 bis 8, dadurch gekennzeichnet, dass die vorgenannten Besprengungsverteiler und das vorgenannte Gitter durch einen Kreislauf (31, 34) mit methanischer Gärungsflüssigkeit, die in dem Abteil (5) enthalten und mit in einem Schacht (17) enthaltenen Gülle vermischt ist, versorgt werden.

## Claims

1. Method of producing methane from vegetable and/or animal wastes in liquid and/or solid form and of the kind consisting in disposing the said wastes into at least one container where they undergo an aerobic fermentation before being dipped and displaced inside of an anaerobic fermentation enclosure containing a methanic fermentation liquid, characterized in that at least the upper portion of the container is kept flush with the level of the aforesaid methanic fermentation liquid during at least one part of the way of the containers within the anaerobic fermentation enclosure.

2. Plant for carrying out the method according to claim 1 and of the type comprising a tank (1) adapted to receive vegetable and/or animal wastes previously disposed into at least one container (37, 51) and undergoing within a compartment (2) of this tank an aerobic fermentation followed by an anaerobic fermentation within another compartment (5) where the said containers are moving, characterized in that the compartment (5) for anaerobic fermentation is provided inside with a conveyor (45, 46, 53) for carrying along the containers (37, 51) towards a position which is flush with the surface (30) of the methanic fermentation liquid contained within the said compartment (5).

3. Plant according to claim 2, characterized in that in the case where the containers are parallelepipedic boxes (37), the aforesaid conveyor consists of a chain (46) associated with a sinuous ramp (45) which extends within the aforesaid compartment (5) as well as within the compartment (6) for discharging the boxes from the tank (1).

4. Plant according to claim 2 or 3, characterized in that the boxes (37) are provided on two opposite sides with lugs (43) coming into engagement with the chain (46) and guided in grooves or slide ways (42) provided on the walls of the aerobic and anaerobic fermentation compartments (2, 5).

5. Plant according to one of claims 2 to 4, characterized in that distributers (48) for spraying the surface (30) of the aforesaid liquid are provided between the said surface and the top wall (12) of the anaerobic fermentation compartment (5).

6. Plant according to claim 2, characterized in that in the case where the containers are perforated bags (51) of plastics material, a conveyor (53) for transporting these bags is arranged at the lower portion of the aforesaid compartment (5) whereas at the upper portion of this compartment is provided a grid (55) retaining the bags in flush relationship with the surface (30) of the fermentation liquid and forming a spraying rack.

7. Plant according to claim 6, characterized in that the aforesaid grid (55) may be replaced by a conveyor retaining and transporting the bags (51) in flush relationship with the surface (30) of the fermentation liquid whereas a spraying rack is provided above the said conveyor.

8. Plant according to claim 6 or 7, characterized in that a supply (56) of impelled or high pressure liquid opens into the bottom wall of the anaerobic fermentation compartment (5) for urging the bags (51) upwards of this compartment.

9. Plant according to one of claims 2 to 8, characterized in that the spraying racks and the grid aforesaid are fed by a circuit (31, 34) with methanic fermentation liquid contained within the compartment (5) and mixed with liquid manure contained in a pit (17).

**Fig.1**

Fig. 2